# EUROPEAN PATENT APPLICATION

(11) **EP 4 360 903 A1**
(43) Date of publication of application: **01.05.2024**
(21) Application number: 22204028.9
(22) Date of filing: 27.10.2022
(51) Int. Cl.: B43L 13/02

(54) **DRAWING SYSTEM**

(71) Applicant: BIC Violex Single Member S.A., 145 69 Anoixi (GR)
(72) Inventor: Galanis, Christos, 145 69 Anoixi (GR)
(74) Representative: Peterreins Schley

(57) **Abstract**

The present disclosure is related to a smart drawing system. The smart drawing system comprises a drawing unit comprising a drawing instrument, wherein the drawing unit comprises a detectable component configured to be detected by one or more sensors, wherein the drawing unit is configured to move the drawing instrument along a third axis between a retracted position and non-retracted position and wherein ink is applied to a target surface when the drawing instrument is at the non-retracted position, wherein the target surface extends along a first axis and a second axis and forms an angle with the third axis, and a memory configured to store an image, a sensor device configured to collect data relating to a position of the drawing unit through one or more sensors and configured to output the data to the drawing unit when the drawing unit is moved by a user such that a drawing tip of the drawing unit facing the target surface is in contact with the target surface, and wherein the drawing unit determines when to move the drawing instrument to the non-retracted position or to the retracted position on the basis of the data relating to the position of the drawing unit and the image stored in the memory.

## Description

### TECHNICAL FIELD

The present disclosure relates to a smart drawing system, an associated computer-implemented method for drawing an image on a target surface using the smart drawing system, and a method for using the smart drawing system.

### BACKGROUND

Tattoos are becoming more and more popular. Since permanent tattoos are a good decision to consider, as they are difficult to remove and many people are not willing to do so, the trend is towards temporary tattoos, which can be applied to skin with a suitable ink and wear off by itself after some time or can be removed by washing it off. Stencils can be used to apply temporary tattoos to the skin.

Stenciling produces an image or pattern by applying pigment to a surface under an intermediate object with designed gaps in it which create the pattern or image by only allowing the pigment to reach some parts of the surface. Usually, a stencil is understood to mean a thin sheet of material, such as paper, plastic, wood or metal with letters or a design cut from it. Stencils are used to produce the said letters or designs on an underlying surface by applying pigment through the cut-out holes in the material.

Stencils of any kind are especially popular among children. Thereby benefits result from working with stencils. For example, stencils help children to develop fine motor skills, boost eye-hand coordination or increase self-esteem.

On the other hand, templates must be purchased, and they limit the user's options or his/her own creativity. Therefore, the user has to compromise existing and commercially available stencils, wherein finding, ordering, and receiving the desired stencil is very time demanding. This means that the user cannot simply transfer his own artwork onto the skin, or may have to create his own template, which can be very time-consuming. Even if there are different templates available in a large database, they must be converted into a physical template. This requires additional equipment, such as a printer.

Another disadvantage is the lack of sustainability of commercial stencils, which often contain a non-negligible amount of plastic and thus produce unnecessary waste, and stencils are often disposable or only slightly reusable, further exacerbating the waste problem.

A further problem is that freehand drawings on the skin usually do not achieve the desired result without the use of a template so that at least one type of guiding of the pen is necessary.

The preceding findings lead to the situation that users need a quick, agile and creatively immersive way to replicate their own or other creators' artwork. Another need is to curb and reduce the accumulation of waste through existing stencils against the background of the increasing trend of temporary tattoos. A replication procedure that enables their creative skills with necessary guidance and help for successful results would be welcome to the users.

### SUMMARY

According to a first aspect, a smart drawing system is provided. The smart drawing system comprises a drawing unit comprising a drawing instrument, wherein the drawing unit comprises a detectable component configured to be detected by one or more sensors, wherein the drawing unit is configured to move the drawing instrument along a third axis between a retracted position and non-retracted position and wherein ink is applied to a target surface when the drawing instrument is at the non-retracted position, wherein the target surface extends along a first axis and a second axis and forms an angle with the third axis, and a memory configured to store an image, a sensor device configured to collect data relating to a position of the drawing unit through the one or more sensors and configured to output the data to the drawing unit when the drawing unit is moved by a user such that a drawing tip of the drawing unit facing the target surface is in contact with the target surface, and wherein the drawing unit determines when to move the drawing instrument to the non-retracted position or to the retracted position on the basis of the data relating to the position of the drawing unit and the image stored in the memory.

According to a second aspect, a computer-implemented method for printing an image on a target surface using a smart drawing system in accordance with the first aspect is provided. The method comprises receiving an image from a camera or scanner, an image from a database selected by the user via a user interface, or an image digitally drawn on an input medium by the user, preparing the image to be drawn on the target surface such that dimensions of the image matches the dimensions of the sensor device defined by a second axis and a third axis, receiving data relating to a position of the drawing unit and determining location coordinates of the drawing unit relative to a reference point inside or on the sensor device based on the received data, and generating driving signals based on the image and the location coordinates of the drawing unit.

According to a third aspect, a method for using a smart drawing system is provided. The method comprises capturing an image with a camera or digitalizing an image with a scanner, selecting an image from a database, or drawing an image digitally on an input medium;
guiding the drawing unit inside or on the sensor device such that the drawing tip is in contact with the target surface.

An effect of the technique of the present specification is to provide a smart drawing system, that may allow the user to apply an artwork to a target surface, such as human skin. This results in various advantages.

An advantage of the system according to the present disclosure may be that the system is reusable and flexible since no further components or disposable products such as stencils are required. This can significantly reduce the amount of waste, for example in the field of temporary tattoos. Nevertheless, no free hand drawing is required, since the system supports the user in drawing in such a way that quick and precise drawing may be possible. Another advantage is that even inexperienced users or users who have no special drawing skills may achieve a good drawing result with the system and thus be encouraged to practice art. Also, with the help of this system, the user may perform drawings with the non-dominant hand, so that, for example, the user may transfer an artwork on the arm of his dominant hand by using is non-dominant hand. Another advantage is flexibility, since drawings may be selected from a database, for example, or the user's own digitized images may be used to transfer them to a target surface using this system disclosed herein.

Another advantage may be that the user may digitize his own hand drawings, such as those created on paper, and use the system to transfer them to target surfaces where drawing is more difficult than on paper, such as on human skin. Thereby, the present disclosure enables the user to create the motif for the artwork himself or to select it from a collection, or to adapt the selected one to his own wishes. That may enhance the creativity of the user and may allow personalization of the image printed on the stencil. Also, it may be possible to exchange the user's own motifs with other users via the database.

Another advantage of the present disclosure arises from the design of the drawing unit, which is designed in such a way that drawing instrument, e.g., a pen, that is inserted into the drawing unit is interchangeable and thus different colors may be applied to the target surface, or, for example, different thicknesses of the drawn shapes are possible. For example, the type of pen used can also be used to change the amount of ink applied to the target surface per drawing operation. The drawing unit thus allows flexibility in the selection of the pen.

Further, by its design, the system may be attached to different target surfaces, such as different parts of the body, to transfer a drawing to the corresponding skin locations. Also, energy storage operation may allow the system to be used in places where there is no power connection, making the system portable and flexible. Further, the adhesive surface may prevent the sensor device from slipping on the target surface, thus enabling more precise work while the ink is being applied to the target surface.

### BRIEF DESCRIPTION OF THE DRAWINGS

Other characteristics will be apparent from the accompanying drawings, which form a part of this disclosure. The drawings are intended to further explain the present disclosure and to enable a person skilled in the art to practice it. However, the drawings are intended as non-limiting examples. Common reference numerals on different figures indicate like or similar features.
- **Figure 1**: schematically illustrates an example of a smart drawing system.
- **Figure 2-1**: schematically illustrates a retracted position of the drawing instrument.
- **Figure 2-2**: schematically illustrates a non-retracted position of the drawing instrument.
- **Figure 3**: schematically illustrates an example of a drawing unit.
- **Figure 4**: schematically illustrates an example of a sensor device comprising a drawing frame exemplary attached to a human body.
- **Figure 5**: schematically illustrates exemplary steps of a computer-implemented method for drawing an image on a target surface using the smart drawing system.

### DETAILED DESCRIPTION

References throughout the preceding specification to "one embodiment", "an embodiment", "one example" or "an example", "one aspect" or "an aspect" means that a particular feature, structure, or characteristic described in connection with the embodiment or example is included in at least one embodiment of the present disclosure. Thus, appearances of the phrases "in one embodiment", "in an embodiment", "one example" or "an example", "one aspect" or "an aspect" in various places throughout this specification are not necessarily all referring to the same embodiment or example.

Furthermore, the particular features, structures, or characteristics can be combined in any suitable combinations and/or sub-combinations in one or more embodiments or examples.

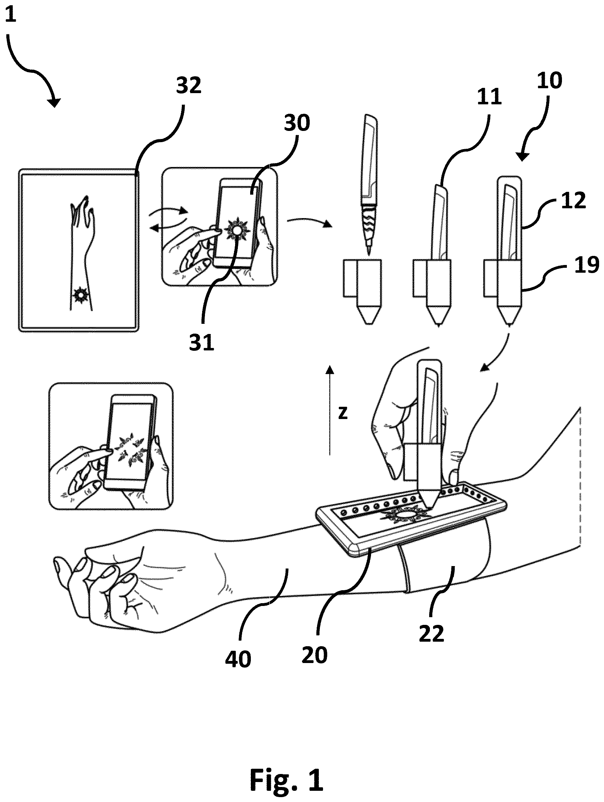

**Fig. 1** schematically illustrates an example of a smart drawing system 1.

According to the first aspect, the smart drawing system comprises a drawing unit 10 comprising a drawing instrument 11, wherein the drawing unit 10 comprises a detectable component 14 configured to be detected by one or more sensors 21a, 21b, wherein the drawing unit 10 is configured to move the drawing instrument 11 along a third axis z between a retracted position A and non-retracted position B and wherein ink is applied to a target surface 40 when the drawing instrument 11 is at the non-retracted position B, wherein the target surface 40 extends along a first axis x and a second axis y and forms an angle with the third axis z, and a memory configured to store an image, a sensor device 20 configured to collect data relating to a position of the drawing unit 10 through the one or more sensors 21a, 21b and configured to output the data to the drawing unit 10 when the drawing unit 10 is moved by a user such that a drawing tip 13 of the drawing unit 10 facing the target surface 40 is in contact with the target surface 40, and wherein the drawing unit 10 determines when to move the drawing instrument 11 to the non-retracted position or to the retracted position A on the basis of the data relating to the position of the drawing unit 10 and the image stored in the memory. The drawing unit 10 may be moved inside an area determined through the sensor device 20 or close to the sensor device 20. In an example, the area may be determined through the dimensions of the sensor device 20 and/or an area configured to be sensed by the one or more sensors 21a, 21b. The target surface 40 may be a planar plane or an approximately planar plane extending along the first axis x and the second axis y. The target surface 40 may also be a curved surface extending in the first axis x and in the second axis y. The curvature may extend along the third axis z, with parallels of the third axis z forming normal vectors on the plane. The target surface 40 may also include a plurality of curvatures, or in other words, the target surface 40 does not have to be a planar surface. In an example, the angle between the first axis x, the second axis y, and the third axis y may be around 90°, in a range from 45° to 90°, in a range from 90° to 120°, in a range from 45° to 120°, <45°, or >120°.

In embodiments, the sensor device 20 may comprise a digital surface (not shown) and the one or more sensors 21a, 21b are arranged in an array along the digital surface. In an example, the one or more sensors 21a, 21b may comprise magnetometers. Such surface is described in US2020166668A1, its content being herein incorporated by reference. The drawing unit 10 may comprise in addition or as the detectable component 14 a magnetic element (not shown) that can be fixed on the drawing unit 10 or on the drawing instrument 11. In examples, the magnetic element may be a magnetic ring. The ring may be fixed on an outer periphery of the body of the drawing instrument 11. In examples, the ring may surround the body that is made of non-magnetic material of the drawing unit 10. The magnetic ring may be of cylindrical shape comprising a cylindrical outer face of circular cross section. The ring may also comprise an outer layer of magnetic material and an inner layer of, for example, elastic material thereby making the magnetic ring deformable upon the introduction of the drawing instrument 11 into the ring without deforming the layer of the magnetic material. In case the one or more sensors 21a, 21b of the digital surface may comprise magnetometers, each magnetometer measures the direction and the intensity of the magnetic field generated by the magnetic element that is fixed on the drawing unit 10.

In that specific embodiment, the target surface may be a sheet of paper. In an example, the drawing unit 10 may be moved on the digital surface. In an example, the target surface may be on the digital surface and the drawing unit 10 may be moved on the digital surface with the target surface in between.

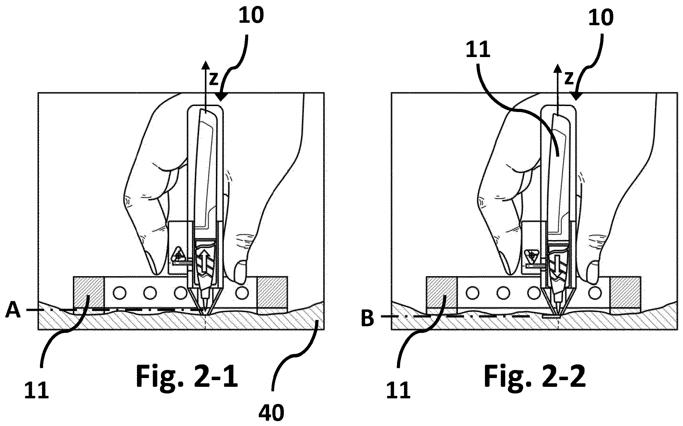

**Fig. 2-1** schematically illustrates a retracted position A of the drawing instrument and **Fig. 2-2** schematically illustrates a non-retracted position B of the drawing instrument. In embodiments, no ink is applied to the target surface 40 when the drawing instrument 11 is at the retracted position A and ink is applied to the target surface 40 when the drawing instrument 11 is at the non-retracted position B. In an example, the sensor device 20 may comprise a drawing frame and the one or more sensors 21a, 21b may be located at an inner side of one or more inner sides of the drawing frame. In an example, the sensor device may span a coordinate system. In an example, the free area within the drawing frame may be part of a coordinate system spanned by the sides of the drawing frame. In an example, the digital surface may correspond to a coordinate system spanned by the sides of the digital surface. The position of the drawing unit 10 corresponds to coordinates inside the coordinate system of the sensor device 20 wherein the coordinates may be formed by the first axis x and the second axis y defined by the coordinate system of the sensor device 20. In an example, a position (e.g., determined through x and y coordinates) on the target surface 40 may correspond to a position (e.g., determined through x and y coordinates (location coordinates)) within the coordinate system of the sensor device 20. If the drawing unit 10 is located at a coordinate or position within or on the sensor device 20 where, according to the image, ink must be applied to the target surface 40, for example because a line or point is drawn at the same position in the image, the drawing unit 10 determines to move the drawing instrument 11 to a non-retracted position B. If the drawing unit 10 is located at a coordinate or position within or on the sensor device 20 where, according to the image, no ink needs to be applied to the target surface 40, for example in the case of free areas or if there are no contours at the respective position, the drawing unit 10 determines to move the drawing instrument 11 to a retracted position A.

In embodiments, at least one of the one or more sensors 21a, 21b may be a magnetic field sensor and the detectable component may generate a magnetic field configured to be detected by the magnetic field sensor.

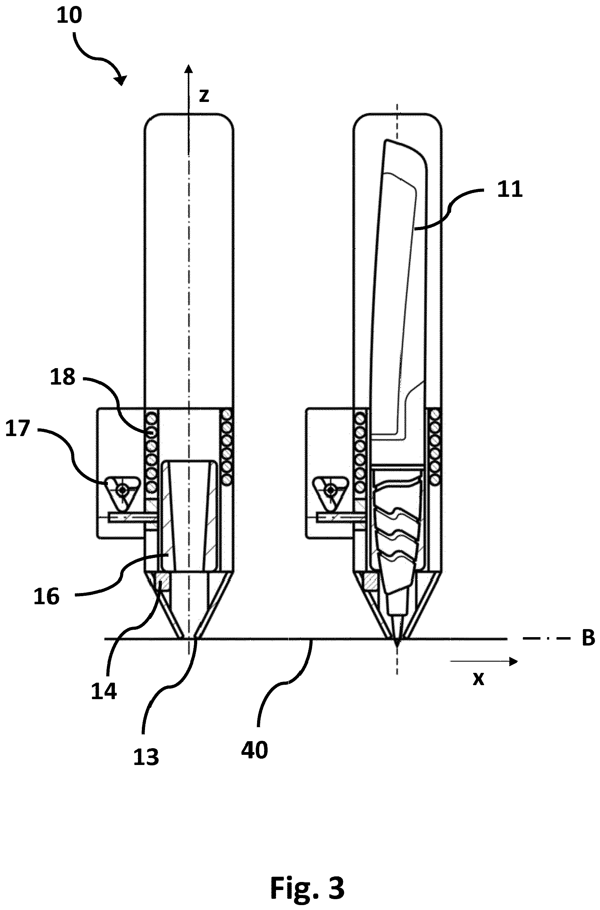

**Fig. 3** schematically illustrates an example of a drawing unit 10 with an exemplary location of the detectable component. In an example, the detectable component 14 may be arranged adjacent to the drawing tip 13 of the drawing unit 10. Ink is applied to the target surface 40 at the position of the drawing tip 13 when the drawing instrument 11 is in a non-retracted position B. To increase the positioning accuracy of the drawing tip 13 of the drawing instrument 11, the detectable component may be arranged close to the drawing tip 13 of the drawing instrument 10, e.g., inside the cone forming the cone-shaped termination of the drawing unit 10 as mentioned above. In an example, an offset may be determined corresponding to the location of the detectable component 14 inside the drawing unit 10 to calculate the position of the drawing tip 13 of the drawing unit 10 within or on the sensor device 20 based on the position of the detectable component 14 in the coordinate system of the sensor device 20. In an example, inside the coordinate system of the sensor device 20, the offset may be an x-value if the x-value of the position of the detectable component 14 may not match the x-value of the position of the drawing tip 13. In an example, inside the coordinate system of the sensor device 20, the offset may be a y-value if the y-value of the position of the detectable component 14 may not match the y-value of the position of the drawing tip 13. In an example, the drawing unit 10 and/or the sensor device 20 may have a marker that indicates to the user with which orientation the drawing unit 10 need to be moved within or on the sensor device 20. In an example, the orientation may be a rotation of the drawing unit 10 around the third axis z. In an example, the detectable component 14 may be a ring element along the circumference of the drawing tip 13 and concentrically surrounds the drawing tip 13. For example, the drawing unit 10 may have a plurality of detectable components 14 arranged around the drawing tip 13, with an offset used for each of the detectable components 14 to determine the position of the drawing tip 13. In an example the magnetic field sensor may comprise a 3-axis magnetometer configured to track position and orientation of the drawing unit 10. The 3-axis magnetometer may be configured to track the orientation of the magnetic field of the detectable component 14 related to the first axis x, the second axis y, and/or the third axis z.

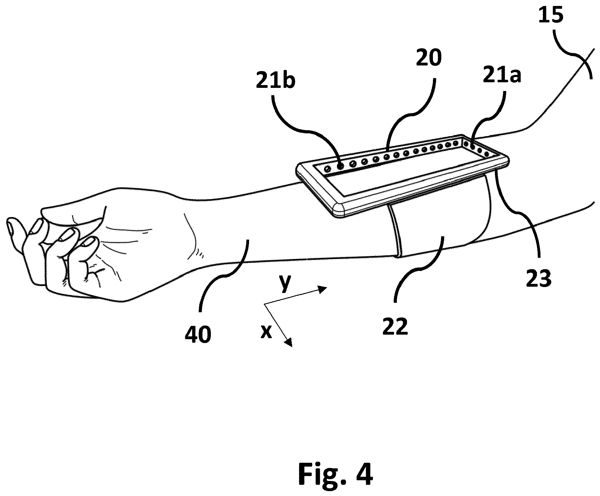

**Fig. 4** schematically illustrates an example of a sensor device 20 comprising a drawing frame exemplary attached to a human body.

For example, the number of the one or more sensors 21a, 21b may be 1, 2, 3, 4, 5, 6, 7, 8, 9, >10, >20, or >100. In case the sensor device 20 comprises a drawing frame, the number of the one or more inner sides of the drawing frame may be 1, 2, 3, 4, 5, 6, or more. In an example, the number of the one or more sensors 21a, 21b is at least two and the at least two sensors 21a, 21b are located at the one or more inner sides with a predetermined distance from each other. In an example, the drawing frame may comprise at least two sensors 21a, 21b and wherein a first sensor 21a of the at least two sensors 21a, 21b may be located at a first inner side of the drawing frame and a second sensor may be located at a second inner side of the drawing frame opposite the second inner side or the first sensor 21a of the at least two sensors 21a, 21b may be located at the first inner side of the drawing frame and the second sensor 21b may be located at a third inner side adjacent to the first inner side, or a combination thereof For example, a first side of the drawing frame may extend along a first axis x, and a second side of the drawing frame, for example, perpendicular to the first side of the drawing frame may extend along a second axis y. For example, one or more sensors disposed on the first side of the drawing frame may correspond to x values of the coordinate system within the drawing frame, and one or more sensors disposed on the second side of the drawing frame may correspond to y values of the coordinate system within the drawing frame. For example, using a first sensor 21a of the at least two sensors 21a, 21b whose position corresponds to an x-value within the coordinate system of the drawing frame and which detects the detectable component 14 and a second sensor 21b whose position corresponds to a y-value within the coordinate system of the drawing frame and which also detects the detectable component 14, the position of the detectable component 14 can be concluded. In an example, the position of the drawing tip 13 of the drawing unit 10 can be determined by means of the offset described above. In an example, the position of the drawing tip 13 may be determined by subtracting or adding the offset in the direction of the first axis x and/or the second axis y. In case the drawing frame have a circular shape, the one or more sensors 21a, 21b may be located across the periphery of the inner circular surface. More than one sensor may be advantageous concerning the tracking (position and/or orientation) accuracy. In an example, the one or more sensors 21a, 21b are embedded in the drawing frame such that the one or more sensors 21a, 21b does not protrude beyond the respective inner side of the one or more inner sides. In an example, the sensor device 20 may be shaped rectangular, square, circular, triangular, polygonal, elliptical, slot shaped, or may have any other geometric shape. In case the sensor device 20 comprises both the drawing frame and the digital surface, the tracking (position and/or orientation) accuracy may be increased.

In examples, the detectable component 14 may be a permanent magnet or an electromagnet. In examples, the detectable component 14 may generate a magnetic field that can be detected by the one or more sensors 21a, 21b. In an example, the detectable component 14 may comprise a hall effect sensor configured to detect the magnetic field generated by the detectable component 14. In an example, the hall effect sensor may generate an output signal indirectly proportional to the distance between the detectable component 14 and the respective sensor of the one or more sensors 21a, 21b. For example, the amplitude of the output signal may be determined by the magnetic field strength and/or magnetic flux density of the magnetic field generated by the detectable component 14. For example, the output signal may have a higher amplitude when the detectable component 14 is closer to the respective sensor, or the output signal may have a lower amplitude when the detectable component 14 is further distanced from the respective sensor. In an example, the output signal may be an electrical voltage/current. In case the sensor device 20 comprises the drawing frame, the one or more sensors 21a, 21b may be located at one or two opposite inner sides of the one or more inner sides extending along the first axis x. In this case, the second coordinate, e.g., y coordinate, can be determined by means of the amplitude of the output signal, which may indicate the distance of the detectable component from the respective sensor which detects the detectable component 14. In an example, the one or more sensors 21a, 21b are located at one or two opposite inner sides of the one or more inner sides extending along the second axis y. In this case, the first coordinate, e.g., x coordinate, can be determined by means of the amplitude of the output signal, which may indicate the distance of the detectable component from the respective sensor which detects the detectable component 14.

In embodiments, at least one of the one or more sensors 21a, 21b may be an electro-optical sensor. For example, an output signal may depend on the distance of the detectable component 14 to the corresponding sensor of the one or more sensors 21a, 21b. For example, the distance may be determined based on the time delay between emitting light and receiving the light reflected at the drawing unit 10. For example, the position of the drawing tip 14 of the drawing unit 10 can be calculated using an offset. The offset can correspond to the radius of the body of the drawing unit 10 if, for example, the opening of the drawing tip 13 is concentric with the body of the drawing unit 10.

In embodiments, the sensor device 20 may further comprise one or more first wireless communication interfaces configured to transmit the data of the one or more sensors 21a, 21b to the drawing unit 10 and/or a mobile device 30. In an example, the data may include position data, distance data, and/or information as to whether a drawing unit 10 has been detected by one or more sensors of the one or more sensors 21a, 21b. For example, the data may include separate information for each sensor of the one or more sensors 21a, 21b as to whether a drawing unit 10 has been detected. For example, matching may be performed in the drawing unit 10, in the mobile device 30, and/or the drawing unit 10 between the sensors that detected the drawing unit 10 and coordinates of the sensor in the coordinate system of the sensor device 20, so that the position of the drawing unit 10 may be determined. In an example, one or more first wireless communication interfaces may be configured to transmit data concerning a plurality of sensors or all sensors of the one or more sensors 21a, 21b. In an example, at least one wireless communication interface of the one or more first wireless communication interfaces may be part of at least one sensor of the one or more sensors 21a, 21b and may be configured to transmit the data of the respective sensor to the drawing unit 10 and/or the mobile device 30. The one or more first wireless communication interfaces may use Bluetooth and/or Wireless Fidelity (Wi-Fi) as a communication protocol or near field communication. In an example, the sensor device 20 may comprise at least a portion of the memory. In an example, the data of the one or more sensors 21a, 21b may be stored in the memory of the sensor device 20 before the data may be taken from the memory and transmitted by means of the one or more first wireless communication interfaces to the drawing unit 10 and/or the mobile device 30. In an example, the memory of the sensor device 20 may be a read-only memory, random-access memory, or a flash memory. In an example, the sensor device 20 may comprise a control circuitry configured to receive signals from the one or more sensors 21a, 21b relating to the position of the drawing unit 10 in the coordinate system of the sensor device 20. In an example, the control circuitry of the sensor device 20 may be configured to store the data in the memory of the sensor device 20 and/or load the data from the memory of the sensor device 20. In an example, the control circuitry of the sensor device 20 may be configured to provide driving signals and/or the data to the one or more first wireless communication interfaces.

In embodiments, the sensor device 20 may comprise an energy storage configured to provide electrical power to the at least one or more sensors 21a, 21b. In an example, the energy storage may comprise a rechargeable battery configured to supply power to the sensor and the one or more first wireless communication interfaces. In example, the energy storage may be permanently installed within the sensor device 20 or may be removable disposed in the sensor device 20.

In embodiments, the sensor device 20 may further comprise a user interface configured to indicate at least the operating state of the drawing system 1. In an example, the operating state may comprise an ON state or an OFF state of the drawing system 1 or the sensor device 20. In an example, the user interface may provide information about the state of charge of the energy storage of the sensor device 20 or indicate when the state of charge of the energy storage of the sensor device 20 is approaching a low power state.

In embodiments, the user interface comprises at least one of a button, a switch, a display device, one or more light emitting diodes, a tactile vibration system, a sound interaction system comprising a speaker and/or microphone configured to allow voice control of the smart drawing system, or a combination thereof. In an example, the user interface may be electrically coupled to the control circuitry of the sensor device 20 and the control circuitry of the sensor device 20 may be configured to receive signals from the user interface and/or may be configured to provide indication signals to the user interface. In an example, the user interface may be used by the user to switch the sensor device 20 on or off. In an example, the user interface may be used to establish a communication channel with the mobile device 30 and/or the drawing unit 10, for example via Bluetooth. For example, the user interface may be used to start, stop, or pause a drawing process. In an example, the user interface may use vibration, visual, or audible signals to warn the user during the drawing process when movements within the sensor device 20 are performed too slowly or too quickly by the user.

In embodiments, the target surface 40 may be human skin and the sensor device 20 may comprise a fixation band 22 that may be configured to be wrapped around a human body part 15 corresponding to the target surface 40 as shown in **Fig. 4****.** In an example, the sensor device 20 may comprise a first surface 23 facing the target surface 40 that consists of or may comprise a layer of a material, such as rubber or plastic with friction-increasing textures that may be configured to increase friction between the first surface 23 and the target surface 40. In an example, the first surface 23 may comprise a layer of a temporary and/or re-usable adhesive such as silicon-based adhesives, polyurethane (PU) gels-based adhesives, dry adhesives, or acrylic adhesives. This may be advantageous to prevent the sensor device 20 from dislocating, for example when a drawing process has already been started and at least parts of an artwork have been transferred to the target surface 40 and may help to stabilize the sensor device 20 against the target surface 40, to eliminate any tracking errors due to an accidental movement of the sensor device 20 during the drawing process.

In embodiments, the drawing unit 10 may comprise a control unit (not shown) configured to receive the data of the one or more sensors 21a, 21b relating to the position of the drawing unit 10 through a second wireless communication interface and configured to determine location coordinates of the drawing unit 10 relative to a reference point within or on the sensor device 20 based on the received data. In an example, the second wireless communication interface may use Bluetooth and/or Wireless Fidelity (Wi-Fi) as a communication protocol or near field communication. The one or more first wireless communication interfaces and the second wireless communication interface may use the same communication protocol. In an example, the data of the one or more sensors 21a, 21b include information about a distance between a respective sensor of the one or more sensors 21a, 21b which has detected the drawing unit 10 and the drawing unit 10 and/or include information about the positions of the one or more sensors 21a, 21b relative to the reference point within or on the sensor device 20, and the control unit is configured to determine the location coordinates of the drawing unit 10 based on the information about the distance between the respective sensor of the one or more sensors 21a, 21b which has detected the drawing unit 10 and the drawing unit 10 and/or the information about the positions of the one or more sensors 21a, 21b which have detected the drawing unit 10 within or on the sensor device 20 as explained above. In an example, the distance between the one or more sensors 21a, 21b and the drawing unit 10 may be determined by means of the magnetic field of the detectable component 14 as explained above. In an example, the drawing unit 10 may comprise at least a portion of the memory configured to store at least the data relating to the position of the drawing unit 10 and the image. In an example, the memory may be a read-only memory, a random-access memory, or a flash memory. In an example, drawing unit 10 may comprise an energy storage configured to provide electrical power to at least the control unit. In an example, the energy storage may comprise a rechargeable battery configured to supply power to the components of the drawing unit 10, at least the control unit. In an example, the energy storage may be permanently installed within the drawing unit 10 or may be removable disposed in the drawing unit 10.

In embodiments, the control unit may be configured to receive the image 31 via the second wireless communication interface from the mobile device 30 and may be configured to store the image 31 in the memory. **Fig. 1** exemplary illustrates the mobile device 30 showing the image 31. In an example, the image 31 may be stored in a database, wherein the mobile device may serve as a communication bridge to the drawing unit 10. The mobile device 30 may be used by the user to select the image 31 out of a plurality of images stored in the database. In an example, a plurality of images may be stored in the mobile device 30. In an example, a software application may be operated on the mobile device 30 providing a plurality of images stored in the database or in the mobile device 30 for selection by the user. In an example, the image 31 may be a photograph taken by means of the mobile device 30 or by means of a camera.

In embodiments, the control unit generates driving signals based on the image 31 and the location coordinates of the drawing unit 10 relative to the reference point within or on the sensor device 20. In an example, the location coordinates may be a combination of a x-coordinate and a y-coordinate of the position of the drawing unit 10 within or on the sensor device 20 related to the reference point, e.g. the origin of the coordinate system within or on the sensor device 20. In an example, the image 31 may be placed in a coordinate system. In an example, the coordinate system of the sensor device 20 may match the coordinate system of the image (e.g., both coordinate system may have the same reference point in a global coordinate system) or may be offset to the coordinate system of the image (e.g., both coordinate systems may have different reference points within a global coordinate system). In an example, the location coordinates of the drawing unit 10 may be matched with the coordinate system of the image 31. Based on the matching, the driving signals may be determined. In an example, the location coordinates of the drawing unit 10 can be used to determine one or more pixels that are located in the position in the image 31 corresponding to the location coordinates of the drawing unit 10 within or on the sensor device 20. In an example, the driving signals may be determined from the brightness values and/or the color values of the one or more pixels located in the position in the image 31 corresponding to the location coordinates of the drawing unit 10 within or on the sensor device 20. In an example, the driving signals may serve to move the drawing instrument 11 into a non-retracted position B or into a retracted position A. For example, at high brightness values of the corresponding one or more pixels driving signals may be generated to move the drawing instrument into a retracted position A. For example, at low brightness values of the corresponding one or more pixels, driving signals may be generated to move the drawing instrument to a non-retracted position. For example, an image may comprise multiple colors. In an example, for positions of the drawing unit 10 corresponding to pixels having a color corresponding to the color of the ink of the drawing instrument 11, driving signals may be generated to move the drawing instrument 11 to a non-retracted position B. In an example, for positions of the drawing unit 10 corresponding to pixels having a color that do not correspond to the color of the ink of the drawing instrument, driving signals may be generated to move the drawing instrument to a retracted position A. In an example, the control unit may comprise a processor. In an example, the processor may perform the matching of the coordinate systems and/or the location coordinates of the drawing unit 10 with the image, and/or the determination of the brightness values and/or color values and may determine the driving signals therefrom. The processor may further comprise a multi-core processor.

In embodiments, the drawing unit 10 may further comprise a moving mechanism 17, wherein the moving mechanism 17 may be mechanically coupled to the drawing instrument 11 via a gripper element 16 and may be configured to move the drawing instrument 11 linearly along the third axis z between the retracted position A and the non-retracted position B based on the driving signals from the control unit. **Fig. 2-1** shows the retracted position A of the drawing unit 11 when no ink is applied to the target surface 40. **Fig. 2-2** shows the non-retracted position B of the drawing unit 11 when ink is applied to the target surface 40. **Fig. 3** shows exemplary the gripper element 16 inside the drawing unit 10. In general, the term "between" may include the retracted position A and the non-retracted position B itself, respectively.

In embodiments, the moving mechanism 17 may comprise a linear motor, or a DC or AC motor in combination with one of a screw-and-nut mechanism, a slider-crank mechanism, or a rack-and-pinion mechanism. The moving mechanism may be configured to transform a rotary motion, e.g., of a DC or AC motor, into a linear motion to move the gripper element towards the target surface 40 or away from the target surface 40 along the third axis z. In an example, the drawing unit 10 may comprise a return spring 18 configured to apply a force to the drawing instrument 11 when the drawing instrument 11 is in the non-retracted position B, and wherein the force may be the spring force of the spring acting along the third axis z in the direction of the retracted position A. In an example, the moving mechanism 17 may exert a force against the spring force when the moving mechanism 17 moves the drawing instrument 11 towards the target surface 40 or the non-retracted position B, thus stretching the return spring 18. In an example, the return spring 18 may serve to support the moving mechanism 17 to move the drawing instrument from the non-retracted position B back to the retracted position A. In an example, the moving mechanism 17 may be in a released state without applying a force to the drawing instrument 11 when the return spring 18 may be configured to move the drawing instrument 11 from the non-retracted position back B to the retracted position A. In an example, the return spring 18 may be compressed when the drawing instrument 11 is moved from the non-retracted position B to the retracted position A by the moving mechanism 17 and apply a force toward the non-retracted position B when the moving mechanism 17 is in a released state to move the drawing instrument 11 towards the non-retracted position B.

In embodiments, the gripper element 16 may comprise a surface facing the outer surface of the drawing instrument 11 and comprising rubber or comprising any suitable texture allowing friction to be established between the drawing instrument 11 and the gripper element 16. In an example, the gripper element 16 may be movable arranged inside the drawing unit 10 and may be configured to transfer the linear movement generated by the moving mechanism 17 to the drawing instrument 11. In an example, the gripper element 16 may be linearly (along the third axis z) movable within a guide portion inside the drawing unit 10 and may be mechanically coupled to the moving mechanism 17. In an example, the gripper element 16 may allow the drawing instrument 11 to be removed or replaced by the user with a second drawing instrument, such as one having a second color other than the color of the first drawing instrument 11. In an example, the gripper element 16 may be configured to exert a pressure force on the outer surface of the drawing instrument 11. In an example, the gripper element 16 may comprise a clamping device and/or a screw-clamp device that applies a force to the drawing instrument 11 such that the linear motion of the moving mechanism 17 is transferred to the drawing instrument 11.

In embodiments, the drawing unit 10 may comprise a body 19 with a closable opening through which the drawing instrument 11 can be removed and/or exchanged by the user. In an example, the shape of the drawing unit 10 may correspond to that of a drawing instrument or a stylus but may be designed to enclose inside a conventional drawing instrument. In an example, the drawing unit 10 may comprise the body 19 having a rectangular, square, circular, triangular, polygonal, or elliptical cross-section, or a combination thereof. For example, the drawing unit 10 may comprise a cone-shaped termination at the end of the body 19 facing the target surface 40. In an example, the cone-shaped termination may be terminated by a drawing tip 13. The drawing tip 13 may comprise a hole facing the target surface 40. In an example, the body 19 of the drawing unit may have at least partially a circular cross-section. In an example, the hole of the drawing tip 13 may be concentric with the circular cross-section and/or the base of the cone forming the cone-shaped termination. The inked tip of the drawing instrument 11 may pass through the hole of the tip 13 when the drawing instrument 11 is in a non-retracted position B to apply ink to the target surface 40. The drawing instrument 11 may be one of a ballpoint pen, a rollerball pen, a felt-tip pen, a gel pen, a marker, pencil, a digital pen, a smart pen, a tattoo machine including a tattoo needle, pens/markers with erasable ink, such as pilot frixion pens or thermoresistive inks, or stylus pen. In an example, the drawing instrument may be a marker for skin which may be suitable for temporary tattoos, such as BIC BodyMark^{®} or Freehand Tattoo Marker. In an example, the ink may be black or of any other color. In an example, the ink may be erasable from the target surface 40. In an example, the ink may be based on genipin. In an example, the target surface 40 may be human skin, paper, or a digital screen display. **Fig. 1** shows an example of the drawing unit 10 into which the drawing instrument 11 can be inserted. In an example, the opening may be closable by a cover 12. In an example, the cover 12 may be configured to enclose the drawing instrument 11 when the cover 12 is attached to the body 19 as shown in **Fig. 1****.** In an example, the cover 12 and/or the body 19 of the drawing unit 10 may comprise at least one material selected from plastic, metal, glass, carton, composite, wherein the material may be at least partially opaque and/or transparent.

In embodiments, the drawing unit 10 may further comprise a user interface configured to indicate at least the operating state of the drawing system 1. In an example, the operating state may comprise an ON state or an OFF state of the drawing system 1 or the drawing unit 10. In an example, the user interface may provide information about the state of charge of the energy storage of the drawing unit 10 or may indicate when the state of charge of the energy storage of the drawing unit 10 is approaching a low power state.

In embodiments, the user interface may comprise at least one of a button, a switch, a display device, one or more light emitting diodes, a tactile vibration system, a sound interaction system comprising a speaker and/or microphone configured to allow voice control of the smart drawing system, or a combination thereof. In an example, the user interface may be electrically coupled to the control unit and the control unit may be configured to receive signals from the user interface and/or may be configured to provide indication signals to the user interface. In an example, the user interface may be used by the user to switch the drawing unit 10 on or off. In an example, the user interface may be used to establish a communication channel with the mobile device 30 and/or the sensor device 20, for example via Bluetooth. For example, the user interface may be used to start, stop, or pause a drawing process. In an example, the user interface may use vibration, visual, or audible signals to warn the user during the drawing process when movements within or on the sensor device 20 are performed too slowly or too quickly by the user. In an example, the user interface may indicate to the user when a drawing process has been completed because the image has been fully transferred to the target surface 40. In an example, the user interface may indicate to the user that the user needs to replace the drawing instrument 11 with another drawing instrument 11, for example, to draw a different color of the image 31.

According to the second aspect, the computer-implemented method 100 for printing an image 31 on a target surface using a smart drawing system 1 according to the first aspect described above comprises receiving 110 an image 31 from a camera or scanner, an image from a database 32 selected by the user via a user interface, or an image digitally drawn on an input medium by the user, preparing 120 the image 1 to be drawn on the target surface 40 such that dimensions of the image matches the dimensions of the sensor device 20 defined by a first axis x and a second axis y, receiving 130 data relating to a position of the drawing unit 10 and determining 140 location coordinates of the drawing unit 10 relative to a reference point inside or on the sensor device 20 based on the received data, and generating 150 driving signals based on the image 31 and the location coordinates of the drawing unit 10. In an example, preparing 120 may further comprise using image processing tools to remove unwanted background and/or noise, to adjust color, or smoothing lines. In an example, preparing 120 may further comprise performing color or image segmentation by means of, e.g., (but not limiting) applying k-means algorithm, binarization (thresholding), motion and interactive clustering, compression-based methods, histogram-based methods, edge detection, region-growing methods, or model-based segmentation. As explained above, matching may be performed between the location coordinates of the drawing unit 10 in the coordinate system of the sensor device 20 and the corresponding one or more pixels associated with a position in the coordinate system of the image 31. The coordinate system of the sensor device 20 and the coordinate system of the image may have the same reference point or origin in a global and common coordinate system. In an example, the coordinate system of the sensor device 20 and the coordinate system of the image 31 may be offset from each other in the global coordinate system. In one example, a translation of the location coordinates of the drawing unit 10 may be performed to match the coordinates in the coordinate system of the image 31.

In embodiments, the driving signals may allow at respective location coordinates in accordance with corresponding areas of the image 31 a drawing instrument 11 of the smart drawing system 1 to be moved to a retracted position A such that no ink is applied to the target surface 40 or to a non-retracted position B such that ink is applied to the target surface 40. In an example, the image 31 may be formed by a plurality of portions each having a respective color and the driving signals may allow the drawing instrument 11 to draw a first portion of the plurality of portions having a respective first color that corresponds to the color of the ink of a first drawing instrument 12 before the user may exchange the first drawing instrument 12 with a second drawing instrument 12 having a color that corresponds to a second color of a second portion of the plurality of portions of the image 31. In an example, the image 31 may be divided into the plurality of portions each having a respective color by segmentation as described above. In each drawing operation, a portion of the plurality of portions having a respective color may be drawn and then the user may be indicated to replace the drawing instrument 11 with a drawing instrument of another color. Subsequently, another drawing operation is performed. In an example, receiving 110 the image 31 to be drawn on the target surface 40 may comprise selecting 160 the image from the database 32 randomly. In an example, random may be pseudorandom. This may be advantageous for implementing a game for a plurality of users. In an example, a player and his team may imagine and predict the final artwork within a timely range, while the player (the user of the drawing system 1) gradually draws with the drawing system 1. The team that recognizes the fastest the artwork wins. Or the team should draw for a duration of a few seconds and the team should predict what the drawn design finally depicts.

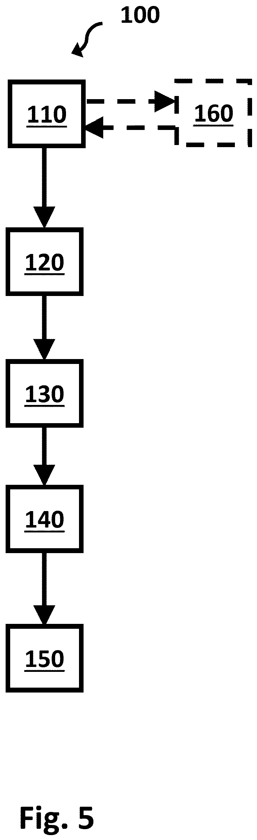

**Figure 5** schematically illustrates exemplary steps of a computer-implemented method for printing an image on a target surface 40 using the smart drawing system 1.

According to the third aspect, the method for using a smart drawing system 1 comprises capturing an image 31 with a camera or digitalizing an image 31 with a scanner, selecting an image 31 from a database 32, or drawing an image 31 digitally on an input medium i.e. a tablet device or a mobile phone device or a laptop or a personal computer device, guiding the drawing unit 10 inside or on the sensor device 20 such that the drawing tip 13 is in contact with the target surface 40. In an example, the method for using the smart drawing system 1 may comprise exchanging or replacing the drawing instrument 11 having a first color with another drawing instrument having a second color. In an example, the drawing system 1 may indicate to the user when to exchange the drawing instrument 11 with another drawing instrument. The user may draw the image digitally on an input medium such as a tablet device or any other suitable computer device which may comprise a touch screen to provide a digital image to be drawn on the target surface 40 using the drawing instrument 1. In an example, the user may control the drawing system 1 via the user interface of the drawing unit 10 and/or the sensor device 20.

References throughout the preceding specification to "one embodiment", "an embodiment", "one example" or "an example", "one aspect" or "an aspect" means that a particular feature, structure, or characteristic described in connection with the embodiment or example is included in at least one embodiment of the present disclosure. Thus, appearances of the phrases "in one embodiment", "in an embodiment", "one example" or "an example", "one aspect" or "an aspect" in various places throughout this specification are not necessarily all referring to the same embodiment or example.

Furthermore, the particular features, structures, or characteristics can be combined in any suitable combinations and/or sub-combinations in one or more embodiments or examples.

### Embodiments:

1. A smart drawing system 1, comprising
   a drawing unit 10 comprising a drawing instrument 11, wherein the drawing unit 10 comprises a detectable component 14 configured to be detected by one or more sensors 21a, 21b, wherein the drawing unit 10 is configured to move the drawing instrument 11 along a third axis z between a retracted position A and non-retracted position B and wherein ink is applied to a target surface 40 when the drawing instrument 11 is at the non-retracted position B, wherein the target surface 40 extends along a first axis x and a second axis y and forms an angle with the third axis z, and
   a memory configured to store an image 31,
   a sensor device 20 configured to collect data relating to a position of the drawing unit 10) through the one or more sensors 21a, 21b and configured to output the data to the drawing unit 10 when the drawing unit 10 is moved by a user such that a drawing tip 13 of the drawing unit 10 facing the target surface 40 is in contact with the target surface 40, and wherein the drawing unit 10 determines when to move the drawing instrument 11 to the non-retracted position or to the retracted position A on the basis of the data relating to the position of the drawing unit 10 and the image 31 stored in the memory.
2. The smart drawing system 1 according to embodiment 1, wherein the sensor device 20 comprises a drawing frame and the one or more sensors 21a, 21b are located at an inner side of one or more inner sides of the drawing frame.
3. The smart drawing system 1 according to embodiment 2, wherein the number of the one or more sensors 21a, 21b is at least two and the at least two sensors 21a, 21b are located at the one or more inner sides of the drawing frame with a predetermined distance from each other.
4. The smart drawing system 1 according to embodiments 2 or 3, wherein the drawing frame comprises at least two sensors 21a, 21b and wherein a first sensor 21a of the at least two sensors 21a, 21b is located at a first inner side of the drawing frame and a second sensor is located at a second inner side of the drawing frame opposite the second inner side or the first sensor 21a of the at least two sensors 21a, 21b is located at the first inner side of the drawing frame and the second sensor 21b is located at a third inner side adjacent to the first inner side, or a combination thereof.
5. The smart drawing system 1 according to any one of embodiments 2, 3, or 4, wherein the one or more sensors 21a, 21b are embedded in the drawing frame such that the one or more sensors 21a, 21b does not protrude beyond the respective inner side of the one or more inner sides.
6. The smart drawing system 1 according to any one of the preceding embodiments, wherein the sensor device 20 comprises a digital surface and the one or more sensors 21a, 21b are arranged in an array along the digital surface.
7. The smart drawing system 1 according to any one of the preceding embodiments, wherein at least one of the one or more sensors 21a, 21b is a magnetic field sensor and the drawing unit 10 comprises a detectable component 14 generating a magnetic field configured to be detected by the magnetic field sensor.
8. The smart drawing system 1 according any one of the preceding embodiments, wherein the sensor device 20 is shaped rectangular, square, circular, triangular, polygonal, elliptical, or slot shaped.
9. The smart drawing system 1 according to any one of the preceding embodiments, wherein the detectable component 14 is a permanent magnet or an electromagnet.
10. The smart drawing system 1 according to any one of the preceding embodiments, wherein at least one of the one or more sensors 21a, 21b is an electro-optical sensor.
11. The smart drawing system 1 according to any one of the preceding embodiments, wherein the sensor device 20 comprises one or more first wireless communication interfaces configured to transmit the data of the one or more sensors 21a, 21b to the drawing unit 10 and/or a mobile device 30.
12. The smart drawing system 1 according to any one of the preceding embodiments, wherein at least one sensor of the one or more sensors 21a, 21b comprises a wireless communication interface configured to transmit the data of the respective sensor to the drawing unit 10 and/or a mobile device 30.
13. The smart drawing system 1 according to any one of the preceding embodiments, wherein the sensor device 20 comprises at least a portion of the memory.
14. The smart drawing system 1 according to embodiment 13, wherein the memory of the sensor device 20 is a read-only memory, random-access memory, or a flash memory.
15. The smart drawing system 1 according to any one of the preceding embodiments, wherein the sensor device 20 comprises an energy storage configured to provide electrical power to the at least one or more sensors 21a, 21b.
16. The smart drawing system 1 according to any one of the preceding embodiments, wherein the target surface 40 is human skin and the sensor device 20 comprises a fixation band 22 that is configured to be wrapped around a human body part 15 corresponding to the target surface 40.
17. The smart drawing system 1 according to any one of the preceding embodiments, wherein the sensor device 20 comprises a first surface 23 facing the target surface 40 that consists of or comprises a layer of a material, such as rubber or plastic with friction-increasing textures, that is configured to increase friction between the first surface 23 and the target surface 40.
18. The smart drawing system 1 according to embodiment 17, wherein the first surface 23 comprises a layer of a temporary and/or re-usable adhesive such as silicon-based adhesives, polyurethane (PU) gels-based adhesives, dry adhesives, or acrylic adhesives.
19. The smart drawing system 1 according to any one of the preceding embodiments, wherein the drawing unit 10 further comprises a control unit configured to receive the data of the one or more sensors 21a, 21b relating to the position of the drawing unit 10 through a second wireless communication interface and configured to determine location coordinates of the drawing unit 10 relative to a reference point within or on the sensor device 20 based on the received data.
20. The smart drawing system 1 according to embodiment 19, wherein the data of the one or more sensors 21a, 21b include information about a distance between a respective sensor of the one or more sensors 21a, 21b which has detected the drawing unit 10 and the drawing unit 10 and/or include information about the positions of the one or more sensors 21a, 21b relative to the reference point within or on the sensor device 20, and the control unit is configured to determine the location coordinates of the drawing unit 10 based on the information about the distance between the respective sensor of the one or more sensors 21a, 21b which has detected the drawing unit 10 and the drawing unit 10 and/or the information about the positions of the one or more sensors 21a, 21b which have detected the drawing unit 10 within or on the sensor device 20.
21. The smart drawing system 1 according to any one of the preceding embodiments, wherein the drawing unit 10 comprises at least a portion of the memory configured to store at least the data relating to the position of the drawing unit 10 and the image data.
22. The smart drawing system 1 according to embodiment 21, wherein the memory is a read-only memory, a random-access memory, or a flash memory.
23. The smart drawing system 1 according to any one of embodiments 19 to 22, wherein the drawing unit 10 comprises an energy storage configured to provide electrical power to at least the control unit.
24. The smart drawing system 1 according to any one of embodiments 19 to 23, wherein the control unit is configured to receive the image 31 via the second wireless communication interface from the mobile device 30 and is configured to store the image 31 in the memory.
25. The smart drawing system 1 according to any one of the embodiments 19 to 24, wherein the control unit generates driving signals based on the image 31 and the location coordinates of the drawing unit 10 relative to the reference point within or on the sensor device 20.
26. The smart drawing system 1 according to any one of embodiments 19 to 25, wherein the control unit comprises a processor.
27. The smart drawing system 1 according to any one of the preceding embodiments, wherein the drawing unit 10 further comprises a moving mechanism 17, wherein the moving mechanism 17 is mechanically coupled to the drawing instrument 11 via a gripper element 16 and is configured to move the drawing instrument 11 linearly along the third axis z between the retracted position A and the non-retracted position B based on the driving signals from the control unit.
28. The smart drawing system 1 according to embodiment 27, wherein the moving mechanism 17 comprises a linear motor, or a DC or AC motor in combination with one of a screw-and-nut mechanism, a slider-crank mechanism, or a rack-and-pinion mechanism.
29. The smart drawing system 1 according to embodiment 27 or 28, wherein the gripper element 16 is movable arranged inside the drawing unit 10 and is configured to transfer the linear movement generated by the moving mechanism 17 to the drawing instrument 11.
30. The smart drawing system 1 according to embodiments 27, 28, or 29, wherein the gripper element 16 comprises a surface facing the outer surface of the drawing instrument 11 and comprising rubber or comprising any suitable texture allowing friction to be established between the drawing instrument 11 and the gripper element 16.
31. The smart drawing system 1 according to any one of embodiments 27 to 30, wherein the gripper element 16 is configured to exert a pressure force on the outer surface of the drawing instrument 11.
32. The smart drawing system 1 according to any one of the preceding embodiments, wherein the drawing unit 10 comprises a return spring 18 configured to apply a force to the drawing instrument 11 when the drawing instrument 11 is in the non-retracted position B, and wherein the force acts along the third axis z in the direction of the retracted position A.
33. The smart drawing system 1 according to any one of the preceding embodiments, wherein the drawing unit 10 comprises a body 19 with a closable opening through which the drawing instrument 11 can be removed and/or exchanged by the user.
34. The smart drawing system 1 according to embodiment 33, wherein the opening is closable by a cover 12.
35. The smart drawing system 1 according to embodiment 33 or 34, wherein the cover 12 and/or the body 19 of the drawing unit 10 comprise at least one material selected from plastic, metal, glass, carton, composite, wherein the material is at least partially opaque and/or transparent.
36. The smart drawing system 1 according to embodiments 33, 34, or 35, wherein the body 19 has a rectangular, square, circular, triangular, polygonal, or elliptical cross-section.
37. The smart drawing system 1 according to any one of the preceding embodiments, wherein the drawing instrument 11 is one of a ballpoint pen, a rollerball pen, a felt-tip pen, a gel pen, a marker, pencil, a digital pen, a smart pen, a tattoo machine including a tattoo needle, pens/markers with erasable ink, such as pilot frixion pens or thermoresistive inks, or stylus pen.
38. The smart drawing system 1 according to any one of the preceding embodiments, wherein the ink is erasable from the target surface 40.
39. The smart drawing system 1 according to any one of the preceding embodiments, wherein the target surface 40 is human skin, paper, or a digital screen display.
40. The smart drawing system 1 according to any one of the preceding embodiments, wherein the drawing unit 10 further comprises a user interface configured to indicate at least the operating state of the drawing system 1.
41. The smart drawing system 1 according to embodiment 40, wherein the user interface comprises at least one of a button, a switch, a display device, one or more light emitting diodes, a tactile vibration system, a sound interaction system comprising a speaker and/or microphone configured to allow voice control of the smart drawing system, or a combination thereof.
42. The smart drawing system 1 according to embodiment 40 or 41, wherein the user interface is electrically coupled to the control unit and the control unit is configured to receive signals from the user interface and/or is configured to provide indication signals to the user interface.
43. A computer-implemented method 100 for drawing an image 31 on a target surface 40 using a smart drawing system 1 according to any one of the preceding embodiments,
   receiving 110 an image 31 from a camera or scanner, an image from a database 32 selected by the user via a user interface, or an image digitally drawn on an input medium by the user;
   preparing 120 the image 31 to be drawn on the target surface 40 such that dimensions of the image matches the dimensions of the sensor device 20 defined by a first axis x and a second axis y;
   receiving 130 data relating to a position of the drawing unit 10 and determining 140 location coordinates of the drawing unit 10 relative to a reference point inside or on the sensor device 20 based on the received data; and
   generating 150 driving signals based on the image 31 and the location coordinates of the drawing unit 10.
44. The computer-implemented method 100 according to embodiment 43, wherein the driving signals allows at respective location coordinates in accordance with corresponding areas of the image 31 a drawing instrument 11 of the smart drawing system 1 to be moved to a retracted position A such that no ink is applied to the target surface 40 or to a non-retracted position B such that ink is applied to the target surface 40.
45. The computer-implemented method 100 according to embodiment 43 or 44, wherein the image 31 is formed by a plurality of portions each having a respective color and the driving signals allows the drawing instrument 11 to draw a first portion of the plurality of portions having a respective first color that corresponds to the color of the ink of a first drawing instrument 12 before the user exchanges the first drawing instrument 12 with a second drawing instrument 12 having a color that corresponds to a second color of a second portion of the plurality of portions of the image 31.
46. The computer-implemented method 100 according to any one of embodiments 43 to 45, wherein receiving 110 the image 31 to be drawn on the target surface 40 comprises selecting 160 the image from the database 32 randomly.
47. A method for using a smart drawing system 1, comprising
   capturing an image 31 with a camera or digitalizing an image 31 with a scanner, selecting an image 31 from a database 32, or drawing an image 31 digitally on an input medium;
   guiding the drawing unit 10 inside or on the sensor device 20 such that the drawing tip 13 is in contact with the target surface 40.

## Claims

1. A smart drawing system (1), comprising
a drawing unit (10) comprising a drawing instrument (11), wherein the drawing unit (10) comprises a detectable component (14) configured to be detected by one or more sensors (21a, 21b), wherein the drawing unit (10) is configured to move the drawing instrument (11) along a third axis (z) between a retracted position (A) and non-retracted position (B) and wherein ink is applied to a target surface (40) when the drawing instrument (11) is at the non-retracted position (B), wherein the target surface (40) extends along a first axis (x) and a second axis (y) and forms an angle with the third axis (z), and
a memory configured to store an image (31),
a sensor device (20) configured to collect data relating to a position of the drawing unit (10) through the one or more sensors (21a, 21b) and configured to output the data to the drawing unit (10) when the drawing unit (10) is moved (20) by a user such that a drawing tip (13) of the drawing unit (10) facing the target surface (40) is in contact with the target surface (40), and wherein the drawing unit (10) determines when to move the drawing instrument (11) to the non-retracted position (B) or to the retracted position (A) on the basis of the data relating to the position of the drawing unit (10) and the image (31) stored in the memory.

2. The smart drawing system (1) according to claim 1, wherein the sensor device (20) comprises a drawing frame and the one or more sensors (21a, 21b) are located at an inner side of one or more inner sides of the drawing frame.

3. The smart drawing system (1) according to any one of claims 1 to 2, wherein the sensor device (20) comprises a digital surface and the one or more sensors (21a, 21b) are arranged in an array along the digital surface.

4. The smart drawing system (1) according to claims 1, 2, or 3, wherein at least one of the one or more sensors (21a, 21b) is a magnetic field sensor and the detectable component (14) generates a magnetic field configured to be detected by the magnetic field sensor.

5. The smart drawing system (1) according to any one of claims 1 to 4, wherein the sensor device (20) comprises one or more first wireless communication interfaces configured to transmit the data of the one or more sensors (21a, 21b) to the drawing unit (10) and/or a mobile device (30).

6. The smart drawing system (1) according to any one of the preceding claims, wherein the sensor device (20) comprises an energy storage configured to provide electrical power to the at least one or more sensors (21a, 21b).

7. The smart drawing system (1) according to any one of the preceding claims, wherein the drawing unit (10) comprises a control unit configured to receive the data of the one or more sensors (21a, 21b) relating to the position of the drawing unit (10) through a second wireless communication interface and configured to determine location coordinates of the drawing unit (10) relative to a reference point within or on the sensor device (20) based on the received data.

8. The smart drawing system (1) according to claim 7, wherein the control unit is configured to receive the image (31) via the second wireless communication interface from the mobile device (30) and is configured to store the image (31) in the memory.

9. The smart drawing system (1) according to claim 7 or 8, wherein the control unit generates driving signals based on the image (31) and the location coordinates of the drawing unit (10) relative to the reference point within or on the sensor device (20).

10. The smart drawing system (1) according to any one of the preceding claims, wherein the drawing unit (10) further comprises a moving mechanism (17), wherein the moving mechanism (17) is mechanically coupled to the drawing instrument (11) via a gripper element (16) and is configured to move the drawing instrument (11) linearly along the third axis (z) between the retracted position (A) and the non-retracted position (B) based on the driving signals from the control unit.

11. The smart drawing system (1) according to claim 10, wherein the moving mechanism (17) comprises a linear motor, or a DC or AC motor in combination with one of a screw-and-nut mechanism, a slider-crank mechanism, or a rack-and-pinion mechanism.

12. The smart drawing system (1) according to any one of the preceding claims, wherein the drawing unit (10) comprises a body (19) with a closable opening through which the drawing instrument (11) can be removed and/or exchanged by the user.

13. The smart drawing system (1) according to any one of the preceding claims, wherein the drawing unit (10) further comprises a user interface configured to indicate at least the operating state of the drawing system (1), wherein the user interface comprises at least one of a button, a switch, a display device, one or more light emitting diodes, a tactile vibration system, a sound interaction system comprising a speaker and/or microphone configured to allow voice control of the smart drawing system (1), or a combination thereof.

14. A computer-implemented method (100) for drawing an image (31) on a target surface (40) using a smart drawing system (1) according to any one of the preceding claims,
receiving (110) an image (31) from a camera or scanner, an image from a database (32) selected by the user via a user interface, or an image digitally drawn on an input medium by the user;
preparing (120) the image (1) to be drawn on the target surface (40) such that dimensions of the image matches the dimensions of the sensor device defined by a first axis (x) and a second axis (y);
receiving (130) data relating to a position of the drawing unit (10) and determining (140) location coordinates of the drawing unit (10) relative to a reference point inside or on the sensor device (20) based on the received data; and
generating (150) driving signals based on the image (31) and the location coordinates of the drawing unit (10).

15. A method for using a smart drawing system (1), comprising
capturing an image (31) with a camera or digitalizing an image (31) with a scanner,
selecting an image (31) from a database (32), or drawing an image (31) digitally on an input medium;
guiding the drawing unit (10) inside or on the sensor device (20) such that the drawing tip (13) is in contact with the target surface (40).
